# EUROPEAN PATENT SPECIFICATION

(11) **EP 4 294 941 B1**
(45) Date of publication and mention of the grant of the patent: **26.03.2025**
(21) Application number: 21706896.4
(22) Date of filing: 18.02.2021
(51) Int. Cl.: C12Q 1/6806, C12Q 1/6855

(54) **STRUCTURE TO PREVENT THREADING OF NUCLEIC ACID TEMPLATES THROUGH A NANOPORE DURING SEQUENCING**
STRUKTUR ZUR VERHINDERUNG DES EINFÄDELNS VON NUKLEINSÄURETEMPLATES DURCH EINE NANOPORE WÄHREND DER SEQUENZIERUNG
STRUCTURE POUR EMPÊCHER L'ENFILAGE DE MATRICES D'ACIDE NUCLÉIQUE À TRAVERS UN NANOPORE PENDANT LE SÉQUENÇAGE

(43) Date of publication of application: 27.12.2023
(73) Proprietor: F. Hoffmann-La Roche AG, 4070 Basel (CH); Roche Diagnostics GmbH, 68305 Mannheim (DE)
(72) Inventor: FRANKLIN, Helen, Pleasanton, California 94588 (US); KLUGHERZ, Spencer, Pleasanton, California 94588 (US); FRIDLAND, Stanislav, Pleasanton, California 94588 (US)
(74) Representative: Göhring, Frank
(86) International application number: PCT/EP2021/053946
(87) International publication number: WO 2022/174898

(56) References cited:
- WO-A1-2015/104302
- WO-A1-2017/203269
- WO-A1-2018/031588
- WO-A1-2018/175997
- WO-A1-2021/078947
- WO-A2-2009/133466
- WO-A2-2021/178893
- US-A1- 2015 118 685
- IMAI KAZUO ET AL: "A novel diagnostic method for malaria using loop-mediated isothermal amplification (LAMP) and MinION(TM) nanopore sequencer", BMC INFECTIOUS DISEASES, vol. 17, no. 1, 1 December 2017 (2017-12-01), XP055851478, Retrieved from the Internet <URL:https://bmcinfectdis.biomedcentral.com/track/pdf/10.1186/s12879-017-2718-9.pdf> DOI: 10.1186/s12879-017-2718-9
- NAGAMINE K ET AL: "Accelerated reaction by loop-mediated isothermal amplification using loop primers", MOLECULAR AND CELLULAR PROBES, ACADEMIC PRESS, LONDON, GB, vol. 16, no. 3, 1 June 2002 (2002-06-01), pages 223 - 229, XP004471001, ISSN: 0890-8508, DOI: 10.1006/MCPR.2002.0415

## Description

### FIELD OF THE INVENTION

The invention relates to the field of nucleic acid sequencing. More specifically, the invention relates to the field of forming libraries of nucleic acid targets for sequencing.

### BACKGROUND OF THE INVENTION

Nucleic acid sequencing using biological and solid-state nanopores is a rapidly growing field, *see* Ameur, et al. (2019) Single molecule sequencing: towards clinical applications, Trends Biotech., 37:72. In some methods, the nucleic acid template is being threaded through a biological nanopore (US10337060) or a solid-state nanopore (US10288599, US20180038001, US10364507), or a tunneling junction between two electrodes PCT/EP2019/066199 and US20180217083). In other methods, the template is not threaded through nanopore but a detectable moiety (e.g., label or tag) is threaded (US8461854). There are methods to prevent or regulate the rate of threading of the template nucleic acid through nanopore. For example, "speed bumps" are complementary oligonucleotides placed outside the pore (US10400278). A tRNA with its trefoil structure can be attached to the template and interact with a "brake" protein to regulate the rate of the template insertion into the pore (US10131944). Hairpins and loops can be present in primers or complementary probes can prevent threading altogether in a non-threading nanopore sequencing method (US9605309). A translating enzyme such as a helicase can also be used to regulate the rate of threading (US20180201993). In solid-state sequencing the template nucleic acid is threaded through a pore in a thin solid layer. Such threading can be regulated with a magnetic bead that "stretches" the nucleic acid strand (US20190317040).

Both Kazuo et al. (BMC Infect Dis. 2017 Sep 13;17(1):621) and Nagamine et al. (Mol Cell Probes. 2002 Jun;16(3):223-9) are concerned with amplicons characterized by stem loops at the termini and the displacement of said loops during extension reactions. None of the various nucleic acid structures in these documents, in particular those presented in Nagamine *et al.,* comprise a 5'-end which upon displacement by the extended 3'-end forms a stem-loop structure. The only nucleic acids with a strand displacement by extension are those used in the cycling amplification step of the LAMP method. These nucleic acids are "dumbbell-like structures" consisting of one strand having stem-loop structures at both ends. During LAMP amplification, the stem-loop at one end of the dumbbell, for example at the 3'-end, is extended and displaces the stem-loop structure at the other end, for example at the 5'-end, eventually resulting in a hairpin-like structure that serves as template for the subsequent elongation steps.

WO 2017/203269 A1 discloses nanopore sequencing methods wherein two or more target polynucleotides are sequentially attached together to form a concatenated polynucleotide. The binding of a second target polynucleotide to a first polynucleotide occurs selectively in the sense that binding only occurs as the first target polynucleotide moves through the pore. As such, the time taken for the second target polynucleotide to contact the pore is reduced and binding of the target polynucleotides present in a sample in the absence of the pore is avoided.

There is a need for innovative and economic means of controlling or preventing threading of nucleic acids though a biological or solid-state nanopore during sequencing. Ideally, the means would add the smallest number of steps or components to a complex sequencing workflow.

### SUMMARY OF THE INVENTION

The invention relates to the use of a double-hairpin adaptor with a self-priming ability. Especially advantageous for nanopore sequencing, the 5'-hairpin prevents threading of the displaced strand into a nanopore. The invention also includes a method of making a library for nucleic acid sequencing and a control nucleic acid molecule for nanopore sequencing.

A target nucleic acid ligated to the novel double-hairpin adaptor so that (at one or both ends), both 5'- and 3'-termini of the resulting nucleic acid construct contain hairpin structures. The 3'-hairpin has an extendable end, which acts as a sequencing primer for the first strand. The second strand is displaced during primer extension but both first and second strands retain their hairpins, which prevent threading into a nanopore.

In some embodiments, the invention is an adaptor for nucleic acid libraries comprising a first strand and a second strand wherein: the first strand has a 5'-portion and a 3'-portion, wherein the 5' portion forms a stem-loop structure having a loop and a stem containing the 5'-end of the first strand, and the 3'-portion comprises a sequence complementary to the second strand; the second strand has a 5'-portion and a 3'-portion, wherein the 3' portion forms a stem-loop structure having a loop and a stem containing the 3'-end of the second strand, and the 5'-portion comprises a sequence complementary to the first strand; and the first and second strands form a duplex via the 3'-portion of the first strand and the 5'-portion of the second strand. The 3'-portion of the second strand may be extendable by a nucleic acid polymerase. The loop-forming regions may be least 4, 5, 6 and up to 20 or more nucleotides long. The adaptor may comprise one or more molecular barcodes selected from a sample barcode (SID) and a unique molecular identifier barcode (UID). For example, the SID can be located outside the duplex formed by the 3'-portion of the first strand and the 5'-portion of the second strand or the UID can be located within the duplex formed by the 3'-portion of the first strand and the 5'-portion of the second strand. The SID and the UID can comprise a predefined sequence or the UID can be a random sequence.

In some embodiments, the invention is a method of making a library of nucleic acids comprising: attaching to a plurality of double-stranded nucleic acids in a sample a plurality of adaptors, each adaptor comprising the first strand having a 5'-portion and a 3'-portion, wherein the 5' portion forms a stem-loop structure having a loop and a stem containing the 5'-end of the first strand, and the 3'-portion comprises a sequence complementary to the second strand; the second strand having a 5'-portion and a 3'-portion, wherein the 3' portion forms a stem-loop structure having a loop and a stem containing an extendable 3'-end of the second strand, and the 5'-portion comprises a sequence complementary to the first strand; and the first and second strands forming a duplex via the 3'-portion of the first strand and the 5'-portion of the second strand. The adaptor may be attached via ligating the duplex formed the 3'-portion of the first strand and the 5'-portion of the second strand to one or both ends of the double-stranded nucleic acids. In some embodiments, prior to attaching, the plurality of nucleic acids is pre-treated to form a blunt end at one or both ends of each nucleic acid. In some embodiments, the duplex formed by the 3'-portion of the first strand and the 5'-portion of the second strand has a single stranded overhang of one or more nucleotides.

In some embodiments, the invention is a method of sequencing nucleic acids in a sample, the method comprising forming a library of nucleic acids as described herein and sequencing the nucleic acids by a sequencing by synthesis method comprising extending the extendable 3'-end of the second strand of the adaptor.

In some embodiments, the invention is a control nucleic acid for use in a sequencing reaction comprising a first strand and a second strand complementary to the first strand and two termini, wherein at least one or the two termini comprises: a 3'-overhang forming a stem-loop structure, wherein the 3'-end of the overhang is extendable by a nucleic acid polymerase, and a 5'-end which upon displacement by the extended 3'-end, forms a stem-loop structure.

In some embodiments, the invention is a method of sequencing a library of nucleic acids comprising contacting the library with a control nucleic acid described herein and sequencing the library of nucleic acid by a method including detection with a nanopore.

### BRIEF DESCRIPTION OF THE DRAWINGS

Figure 1 is a diagram of the sequencing adaptor with two hairpin loops.
Figure 2 is a diagram of the sequencing adaptor with a single hairpin loop.

### DETAILED DESCRIPTION OF THE INVENTION

### Definitions

Unless defined otherwise, technical and scientific terms used herein have the same meaning as commonly understood by a person of ordinary skill in the art. *See,* Sambrook et al., Molecular Cloning, A Laboratory Manual, 4th Ed. Cold Spring Harbor Lab Press (2012).

The following definitions are provided to facilitate understanding of the present disclosure.

The term "adaptor" refers to a nucleotide sequence that may be added to another sequence in order to import additional elements and properties to that sequence. The additional elements include without limitation: barcodes, primer binding sites, capture moieties, labels, secondary structures.

The term "barcode" refers to a nucleic acid sequence that can be detected and identified. Barcodes can generally be 2 or more and up to about 50 nucleotides long. Barcodes are designed to have at least a minimum number of differences from other barcodes in a population. Barcodes can be unique to each molecule in a sample or unique to the sample and be shared by multiple molecules in the sample. The term "multiplex identifier," "MID" or "sample barcode" refer to a barcode that identifies a sample or a source of the sample. As such, all or substantially all, MID barcoded polynucleotides from a single source or sample will share an MID of the same sequence; while all, or substantially all (*e.g.,* at least 90% or 99%), MID barcoded polynucleotides from different sources or samples will have a different MID barcode sequence. Polynucleotides from different sources having different MIDs can be mixed and sequenced in parallel while maintaining the sample information encoded in the MID barcode. The term "unique molecular identifier" or "UID," refer to a barcode that identifies a polynucleotide to which it is attached. Typically, all, or substantially all (*e.g.,* at least 90% or 99%), UID barcodes in a mixture of UID barcoded polynucleotides are unique.

The term "DNA polymerase" refers to an enzyme that performs template-directed synthesis of polynucleotides from deoxyribonucleotides. DNA polymerases include prokaryotic Pol I, Pol II, Pol III, Pol IV and Pol V, eukaryotic DNA polymerase, archaeal DNA polymerase, telomerase and reverse transcriptase. The term "thermostable polymerase," refers to an enzyme that is stable to heat, is heat resistant, and retains sufficient activity to effect subsequent polynucleotide extension reactions and does not become irreversibly denatured (inactivated) when subjected to the elevated temperatures for the time necessary to effect denaturation of double-stranded nucleic acids. In some examples useful for understanding the invention and not encompassed by the wording of the claims, the following thermostable polymerases can be used: *Thermococcus litoralis* (Vent*,* GenBank: AAA72101), *Pyrococcus furiosus* (Pfu, GenBank: D12983, BAA02362), *Pyrococcus woesii, Pyrococcus* GB-D (Deep Vent, GenBank: AAA67131), *Thermococcus kodakaraensisKODI* (KOD, GenBank: BD175553, BAA06142; *Thermococcussp.* strain KOD (Pfx, GenBank: AAE68738)), *Thermococcus gorgonarius* (Tgo*,* Pdb: *4699806), Sulfolobus solataricus* (GenBank: NC002754, P26811), *Aeropyrum pernix* (GenBank: BAA81109), *Archaeglobus fulgidus* (GenBank: 029753), *Pyrobaculum aerophilum* (GenBank: AAL63952), *Pyrodictium occultum* (GenBank: BAA07579, BAA07580), *Thermococcus* 9 degree Nm (GenBank: AAA88769, Q56366), *Thermococcus fumicolans* (GenBank: CAA93738, P74918), *Thermococcus hydrothermalis* (GenBank: CAC18555), *Thermococcus* sp. GE8 (GenBank: CAC12850), *Thermococcus* sp. JDF-3 (GenBank: AX135456; WO0132887), *Thermococcus* sp. TY (GenBank: *CAA73475), Pyrococcus abyssi* (GenBank: P77916), *Pyrococcus glycovorans* (GenBank: CAC12849), *Pyrococcus horikoshii* (GenBank: NP 143776), *Pyrococcus* sp. GE23 (GenBank: CAA90887), *Pyrococcus* sp. ST700 (GenBank: CAC 12847), *Thermococcus pacificus* (GenBank: AX411312.1), *Thermococcus zilligii* (GenBank: DQ3366890), *Thermococcus aggregans, Thermococcus barossii, Thermococcus celer* (GenBank: DD259850.1), *Thermococcus profundus* (GenBank: E14137), *Thermococcus siculi* (GenBank: DD259857.1), *Thermococcus thioreducens, Thermococcus onnurineus* NA1, *Sulfolobus acidocaldarium, Sulfolobus tokodaii, Pyrobaculum calidifontis, Pyrobaculum islandicum* (GenBank: AAF27815), *Methanococcus jannaschii* (GenBank: Q58295), *Desulforococcus* species TOK, *Desulfurococcus, Pyrolobus, Pyrodictium, Staphylothermus, Vulcanisaetta, Methanococcus* (GenBank: P52025) and other archaeal B polymerases, such as GenBank AAC62712, P956901, BAAA07579)), thermophilic bacteria *Thermus* species (e.g., *flavus, ruber, thermophilus, lacteus, rubens, aquaticus), Bacillus stearothermophilus, Thermotoga maritima, Methanothermus fervidus,* KOD polymerase, TNA1 polymerase, *Thermococcus* sp. 9 degrees N-7, T4, T7, phi29, *Pyrococcus furiosus, P. abyssi, T. gorgonarius, T. litoralis, T. zilligii, T.* sp. GT, *P.* sp. GB-D, KOD, Pfu, *T. gorgonarius, T. zilligii, T. litoralis* and *Thermococcus* sp. 9N-7 polymerases. In some cases, the nucleic acid (*e.g.,* DNA or RNA) polymerase may be a modified naturally occurring Type A polymerase. A further example useful for understanding the invention and not encompassed by the wording of the claims relates to a method wherein a modified Type A polymerase, *e.g.,* in a primer extension, end-modification (*e.g.,* terminal transferase, degradation, or polishing), or amplification reaction, may be selected from any species of the genus *Meiothermus, Thermotoga,* or *Thermomicrobium.* Another example useful for understanding the invention and not encompassed by the wording of the claims generally pertains to a method wherein the polymerase, *e.g.,* in a primer extension, end-modification (*e.g.,* terminal transferase, degradation or polishing), or amplification reaction, may be isolated from any of *Thermus aquaticus* (Taq), *Thermus thermophilus, Thermus caldophilus,* or *Thermus filiformis.* A further example useful for understanding the invention and not encompassed by the wording of the claims generally encompasses a method wherein the modified Type A polymerase, *e.g.,* in a primer extension, end-modification (*e.g.,* terminal transferase, degradation, or polishing), or amplification reaction, may be isolated from *Bacillus stearothermophilus, Sphaerobacter thermophilus, Dictoglomus thermophilum,* or *Escherichia coli.* Another example useful for understanding the invention and not encompassed by the wording of the claims generally relates to a method wherein the modified Type A polymerase, *e.g.,* in a primer extension, end-modification (*e.g.,* terminal transferase, degradation, or polishing), or amplification reaction, may be a mutant *Taq*-E507K polymerase. Another example useful for understanding the invention and not encompassed by the wording of the claims generally pertains to a method wherein a thermostable polymerase may be used to effect amplification of the target nucleic acid.

The term "hairpin" refers to a secondary structure formed by a single strand of nucleic acid comprising at least one double stranded region ("stem") and the region forming the stem is interrupted by a single stranded region "loop." There is no defined size or relative size for the stem and loop regions.

The term "nucleic acid" or "polynucleotide" refers to deoxyribonucleic acids (DNA) or ribonucleic acids (RNA) and polymers thereof in either single- or double-stranded form. Unless specifically limited, the term encompasses nucleic acids containing known analogues of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.,* degenerate codon substitutions), alleles, orthologues, SNPs, and complementary sequences as well as the sequence explicitly indicated.

The term "primer" refers to an oligonucleotide, which binds to a specific region of a single-stranded template nucleic acid molecule and initiates nucleic acid synthesis via a polymerase-mediated enzymatic reaction. Typically, a primer comprises fewer than about 100 nucleotides and preferably comprises fewer than about 30 nucleotides. A target-specific primer specifically hybridizes to a target polynucleotide under hybridization conditions. Such hybridization conditions can include, but are not limited to, hybridization in isothermal amplification buffer (20 mM Tris-HCl, 10 mM (NH₄)₂SO₄), 50 mM KCl, 2 mM MgSO₄, 0.1% TWEEN^{®} 20, pH 8.8 at 25 °C) at a temperature of about 40 °C to about 70 °C. In addition to the target-binding region, a primer may have additional regions, typically at the 5'-poriton. The additional region may include universal primer binding site or a barcode.

The term "sample" refers to any biological sample that comprises nucleic acid molecules, typically comprising DNA or RNA. Samples may be tissues, cells or extracts thereof, or may be purified samples of nucleic acid molecules. The term "sample" refers to any composition containing or presumed to contain target nucleic acid. Use of the term "sample" does not necessarily imply the presence of target sequence among nucleic acid molecules present in the sample. The sample can be a specimen of tissue or fluid isolated from an individual for example, skin, plasma, serum, spinal fluid, lymph fluid, synovial fluid, urine, tears, blood cells, organs and tumors, and also to samples of *in vitro* cultures established from cells taken from an individual, including the formalin-fixed paraffin embedded tissues (FFPET) and nucleic acids isolated therefrom. A sample may also include cell-free material, such as cell-free blood fraction that contains cell-free DNA (cfDNA) or circulating tumor DNA (ctDNA). The sample can be collected from a non-human subject or from the environment.

The term "target" or "target nucleic acid" refer to the nucleic acid of interest in the sample. The sample may contain multiple targets as well as multiple copies of each target.

The term "universal primer" refers to a primer that can hybridize to a universal primer binding site. Universal primer binding sites can be natural or artificial sequences typically added to a target sequence in a non-target-specific manner.

Sequencing nucleic acids using biological or solid state nanopores is a rapidly developing field with many technological solutions becoming available (*see* Ameur, et al. (2019) Single molecule sequencing: towards clinical applications, Trends Biotech., 37:72). One common issue with nanopore sequencing is control of translocation (threading) of the nucleic acid through pore. Some workflows include merely controlling the rate with which the nucleic acid moves through the opening. Solutions include the use of a helicase (US20180201993) or a magnetic particle (US20190317040) to slow down or regulate the rate of translocation. Some structures can be connected to the pore for example, a "speed bump" complementary oligonucleotide (US10400278). Other structures are attached to the template molecule, for example, a tRNA structure attached to the template interacting with a "brake" protein (US10131944) in the pore complex or hairpins present on sequencing primers (US9605309).

Some nanopore sequencing technologies do not involve translocation of a strand through a nanopore. In such technologies, preventing the translocation (threading) altogether is desired. The present invention is suitable for both control and prevention of translocation through the nanopore.

In addition to controlling translocation of the strand being sequenced, a separate problem is the disposal of the complementary strand in the case of a double stranded template. When the double-stranded nucleic acid template is unwound during the sequencing-by-synthesis (SBS) reaction, the non-sequenced strand must be prevented from threading through the nanopore in both threading and non-threading embodiments of nanopore sequencing.

The invention comprises methods and compositions related to forming nucleic acid templates for sequencing and libraries of nucleic acids templates for sequencing. The templates and libraries are suitable for any method of sequencing but have a special advantage for nanopore sequencing.

In one embodiment, the invention is a novel adaptor comprising a double stranded portion for ligation to the nucleic acid to be sequenced. The adaptor further comprises a novel feature of at least one stem-loop or hairpin structure on the end opposite the double stranded portion. In some embodiments, the adaptor has a single stem-loop structure. In other embodiments, the adaptor has two stem-loop structures. The adaptor retains an extendable 3'-end, which can serve as a primer, for example a sequencing primer or an amplification primer. The adaptor may comprise any of the features useful in a sequencing adaptor including barcodes, primer binding sites or capture moieties, e.g., for separation or purification of the adapted nucleic acids. For example, the double stranded portion of the adaptor can comprise a unique molecular barcode (UID) to uniquely mark the adapted nucleic acid or a multiplexing sample barcode (SID) or (MID) to identically mark all the nucleic acids in the sample as coming from the same source. The adaptor may comprise a capture moiety such as biotin for capturing the adapted nucleic acids and separating them from non-adapted nucleic acids. The special advantage of the adaptor is the stem-loop or hairpin structure formed by at least one end of each strand in the adapted nucleic acid. The size of the loop is sufficient to inhibit or prevent translocation of the strand through a nanopore during sequencing. Depending on the nanopore used, the length of the loop-forming sequence can be 4, 5, 6 and up to 20 or more nucleotides in order to form a loop of sufficient size to prevent or inhibit translocation. One of skill in the art is able to determine the length of the loop forming sequence either experimentally or empirically knowing the sequence and secondary, tertiary of quaternary structure of the nanopore forming protein.

In one embodiment, the invention is a method of making a library of adapted nucleic acids for sequencing. The novel adaptor is attached to one or both ends of each sample nucleic acid. The library may be purified or separated from unused adaptors and un-adapted sample nucleic acids.

In yet another embodiment, the invention is a control molecule for sequencing a library of sample nucleic acids. The control molecule has ends with novel structures described herein. In particular, the control nucleic acid molecule comprises a first strand and a second strand complementary to the first strand. The control molecule further comprises two termini, wherein at least one or the two termini comprises: a 3'-overhang forming a stem-loop structure, wherein the 3'-end of the overhang is extendable by a nucleic acid polymerase, and a recessed 5'-end which upon displacement by the extended 3'-end, also forms a stem-loop structure. The size of the loop in the stem-loop structure on both strands is sufficient to inhibit or prevent translocation of the strand through a nanopore during sequencing.

The present disclosure comprises simultaneous isolation and sequencing of target nucleic acids in a sample. In some examples useful for understanding the invention and not encompassed by the wording of the claims, the sample is derived from a subject or a patient. In some examples useful for understanding the invention and not encompassed by the wording of the claims the sample may comprise a fragment of a solid tissue or a solid tumor derived from the subject or the patient, *e.g.,* by biopsy. The sample may also comprise body fluids (*e.g.,* urine, sputum, serum, plasma or lymph, saliva, sputum, sweat, tear, cerebrospinal fluid, amniotic fluid, synovial fluid, pericardial fluid, peritoneal fluid, pleural fluid, cystic fluid, bile, gastric fluid, intestinal fluid, or fecal samples). The sample may comprise whole blood or blood fractions where normal or tumor cells may be present. In some examples useful for understanding the invention and not encompassed by the wording of the claims, the sample, especially a liquid sample may comprise cell-free material such as cell-free DNA or RNA including cell-free tumor DNA or tumor RNA. In some examples useful for understanding the invention and not encompassed by the wording of the claims, the sample is a cell-free sample, *e.g.,* cell-free blood-derived sample where cell-free tumor DNA or tumor RNA are present. In other examples useful for understanding the invention and not encompassed by the wording of the claims, the sample is a cultured sample, *e.g.,* a culture or culture supernatant containing or suspected to contain nucleic acids derived from the cells in the culture or from an infectious agent present in the culture. In some examples useful for understanding the invention and not encompassed by the wording of the claims, the infectious agent is a bacterium, a protozoan, a virus or a mycoplasma.

Target nucleic acids are the nucleic acid of interest that may be present in the sample. Each target is characterized by its nucleic acid sequence. The present invention enables detection of one or more RNA or DNA targets. In some examples useful for understanding the invention and not encompassed by the wording of the claims, the DNA target nucleic acid is a gene or a gene fragment (including exons and introns) or an intergenic region, and the RNA target nucleic acid is a transcript or a portion of the transcript to which target-specific primers hybridize. In some examples useful for understanding the invention and not encompassed by the wording of the claims, the target nucleic acid contains a locus of a genetic variant, *e.g.,* a polymorphism, including a single nucleotide polymorphism or variant (SNP of SNV), or a genetic rearrangement resulting *e.g.,* in a gene fusion. In some examples useful for understanding the invention and not encompassed by the wording of the claims, the target nucleic acid comprises a biomarker, *i.e.,* a gene whose variants are associated with a disease or condition. For example, the target nucleic acids can be selected from panels of disease-relevant markers described in U.S. Patent Application Publ. No. 20160032396 filed on September 10, 2015. Such panels are available as AVENIO ctDNA Analysis kits (Roche Sequencing Solutions, Pleasanton, Cal.) In other examples useful for understanding the invention and not encompassed by the wording of the claims, the target nucleic acid is characteristic of a particular organism and aids in identification of the organism or a characteristic of the pathogenic organism such as drug sensitivity or drug resistance. In yet other examples useful for understanding the invention and not encompassed by the wording of the claims, the target nucleic acid is a unique characteristic of a human subject, *e.g.,* a combination of HLA or KIR sequences defining the subject's unique HLA or KIR genotype. In yet other examples useful for understanding the invention and not encompassed by the wording of the claims, the target nucleic acid is a somatic sequence such as a rearranged immune sequence representing an immunoglobulin (including IgG, IgM and IgA immunoglobulin) or a T-cell receptor sequence (TCR). In yet another example useful for understanding the invention and not encompassed by the wording of the claims, the target is a fetal sequence present in maternal blood, including a fetal sequence characteristic of a fetal disease or condition or a maternal condition related to pregnancy.

In some examples useful for understanding the invention and not encompassed by the wording of the claims, the target nucleic acid is RNA (including mRNA, microRNA, viral RNA). In other examples useful for understanding the invention and not encompassed by the wording of the claims, the target nucleic acid is DNA including cellular DNA or cell-free DNA (cfDNA) including circulating tumor DNA (ctDNA). The target nucleic acid may be present in a short or long form. Longer target nucleic acids may be fragmented. In some examples useful for understanding the invention and not encompassed by the wording of the claims, the target nucleic acid is naturally fragmented, *e.g.,* includes circulating cell-free DNA (cfDNA) or chemically degraded DNA such as the one found in chemically preserved or ancient samples.

In some examples useful for understanding the invention and not encompassed by the wording of the claims a step of nucleic acid isolation is comprised. Generally, any method of nucleic acid extraction that yields isolated nucleic acids comprising DNA or RNA may be used. Genomic DNA or RNA may be extracted from tissues, cells, liquid biopsy samples (including blood or plasma samples) using solution-based or solid-phase based nucleic acid extraction techniques. Nucleic acid extraction can include detergent-based cell lysis, denaturation of nucleoproteins, and optionally removal of contaminants. Extraction of nucleic acids from preserved samples may further include a step of deparaffinization. Solution based nucleic acid extraction methods may comprise salting out methods or organic solvent or chaotrope methods. Solid-phase nucleic extraction methods can include but are not limited to silica resin methods, anion exchange methods or magnetic glass particles and paramagnetic beads (KAPA Pure Beads, Roche Sequencing Solutions, Pleasanton, Cal.) or AMPure beads (Beckman Coulter, Brea, Cal.)

A typical extraction method involves lysis of tissue material and cells present in the sample. Nucleic acids released from the lysed cells can be bound to a solid support (beads or particles) present in solution or in a column, or membrane where the nucleic acids may undergo one or more washing steps to remove contaminants including proteins, lipids and fragments thereof from the sample. Finally, the bound nucleic acids can be released from the solid support, column or membrane and stored in an appropriate buffer until ready for further processing. Because both DNA and RNA must be isolated, no nucleases may be used and care should be taken to inhibit any nuclease activity during the purification process.

In some examples useful for understanding the invention and not encompassed by the wording of the claims, the input DNA or input RNA require fragmentation. In such examples, RNA may be fragmented by a combination of heat and metal ions, e.g., magnesium. In some examples useful for understanding the invention and not encompassed by the wording of the claims, the sample is heated to 85°-94°C for 1-6 minutes in the presence of magnesium. (KAPA RNA HyperPrep Kit, KAPA Biosystems, Wilmington, Mass). DNA can be fragmented by physical means, e.g., sonication, using available instruments (Covaris, Woburn. Mass.) or enzymatic means (KAPA Fragmentase Kit, KAPA Biosystems).

In some examples useful for understanding the invention and not encompassed by the wording of the claims, the isolated nucleic acid is treated with DNA repair enzymes. In some examples useful for understanding the invention and not encompassed by the wording of the claims, the DNA repair enzymes comprise a DNA polymerase which has 5'-3' polymerase activity and 3'-5' single stranded exonuclease activity, a polynucleotide kinase which adds a 5' phosphate to the dsDNA molecule, and a DNA polymerase which adds a single dA base at the 3' end of the dsDNA molecule. The end repair/A-tailing kits are available e.g., Kapa Library Preparation, kits including KAPA Hyper Prep and KAPA HyperPlus (Kapa Biosystems, Wilmington, Mass.).

In some examples useful for understanding the invention and not encompassed by the wording of the claims, the DNA repair enzymes target damaged bases in the isolated nucleic acids. In some examples useful for understanding the invention and not encompassed by the wording of the claims, sample nucleic acid is partially damaged DNA from preserved samples, e.g., formalin-fixed paraffin embedded (FFPET) samples. Deamination and oxidation of bases can result in an erroneous base read during the sequencing process. In some examples useful for understanding the invention and not encompassed by the wording of the claims, the damaged DNA is treated with uracil N-DNA glycosylase (UNG/UDG) and/or 8-oxoguanine DNA glycosylase.

In some examples useful for understanding the invention and not encompassed by the wording of the claims, the invention comprises an amplification step. The isolated nucleic acids can be amplified prior to further processing. This step can involve linear or exponential amplification. Amplification may be isothermal or involve thermocycling. In some examples useful for understanding the invention and not encompassed by the wording of the claims, the amplification is exponential and involves PCR. In some examples useful for understanding the invention and not encompassed by the wording of the claims, gene-specific primers are used for amplification. In other examples useful for understanding the invention and not encompassed by the wording of the claims, universal primer binding sites are added to target nucleic acid e.g., by ligating an adaptor comprising the universal primer binding sites. All adaptor-ligated nucleic acids have the same universal primer binding sites and can be amplified with the same set of primers. The number of amplification cycles where universal primers are used can be low but also can be 10, 20 or as high as about 30 or more cycles, depending on the amount of product needed for the subsequent steps. Because PCR with universal primers has reduced sequence bias, the number of amplification cycles need not be limited to avoid amplification bias.

In some embodiments, the invention utilizes an adaptor nucleic acid composed of two strands with the structure described herein. In some examples useful for understanding the invention and not encompassed by the wording of the claims, the adaptor molecules are *in vitro* synthesized artificial sequences. In other examples useful for understanding the invention and not encompassed by the wording of the claims, the adaptor molecules are *in vitro* synthesized naturally occurring sequences. In yet other examples useful for understanding the invention and not encompassed by the wording of the claims, the adaptor molecules are isolated naturally occurring molecules or isolated non-naturally occurring molecules.

The double-stranded end of the hairpin adaptor may be ligated to the double stranded nucleic acid molecules. The adaptor may be ligated at one or both ends of the double-stranded molecule.

The adaptor oligonucleotide can have overhangs or blunt ends on the terminus to be ligated to the target nucleic acid. In some examples useful for understanding the invention and not encompassed by the wording of the claims, the novel adaptor described herein comprises blunt ends to which a blunt-end ligation of the target nucleic acid can be applied. The target nucleic acids may be blunt-ended or may be rendered blunt-ended by enzymatic treatment (e.g., "end repair."). In other examples useful for understanding the invention and not encompassed by the wording of the claims, the blunt-ended DNA undergoes A-tailing where a single A nucleotide is added to the 3'-end of one or both blunt ends. The adaptors described herein are made to have a single T nucleotide extending from the blunt end to facilitate ligation between the nucleic acid and the adaptor. Commercially available kits for performing adaptor ligation include AVENIO ctDNA Library Prep Kit or KAPA HyperPrep and HyperPlus kits (Roche Sequencing Solutions, Pleasanton, CA). In some examples useful for understanding the invention and not encompassed by the wording of the claims, the adaptor ligated DNA may be separated from excess adaptors and unligated DNA.

In some embodiments, the adaptor contains additional features, e.g., a barcode, an amplification primer binding site or a sequencing primer binding site.

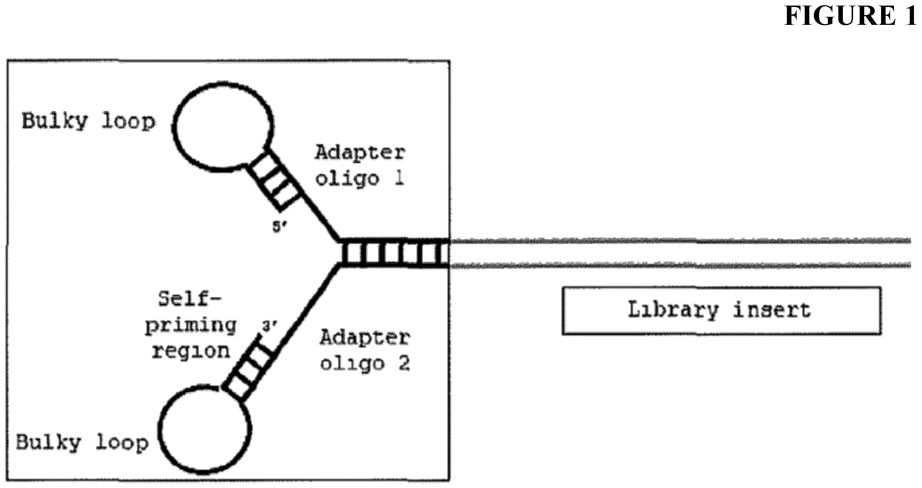

Figure 1 illustrates the novel adaptor ligated to a nucleic acid of interest. Referring to Figure 1, the adaptor comprises a first strand (top strand, "Adapter oligo 1") and a second strand (bottom strand, "Adapter oligo 2"). The adaptor comprises a double-stranded region of hybridization between the first and second adaptor strands ("Adaptor oligos"). This region is capable of being ligated to a target nucleic acid ("library insert"). Each strand also comprises a hairpin region having a stem and a loop. In reference to the adaptor illustrated in Figure 1, the adaptor is ligated to a target nucleic acid on one end and one remaining unligated 5'-end and one remaining unligated 3'-end. The first (top) strand contains the 5'-end, which is non-extendable and is in the double-stranded stem part of the top stem-loop structure. The second (bottom) strand contains the 3'-end. The 3'-end is in the double-stranded stem part of the bottom stem-loop structure. The 3'-end is extendable and can serve as a primer to copy the bottom strand of the target nucleic acid without the need for a separate primer. In this example useful for understanding the invention and not encompassed by the wording of the claims, the 3'-end of the adaptor is a self-priming region. The self-priming region acts as a sequencing primer or an amplification primer.

In some embodiments, the adaptor-ligated nucleic acid is sequenced after adaptor ligation. In other examples useful for understanding the invention and not encompassed by the wording of the claims, the adaptor-ligated target nucleic acid is amplified prior to sequencing. Each one of the copy strands will contain adaptor sequences capable of folding in the double-hairpin structure shown in Figure 1. The size of the loop is sufficient to inhibit or prevent translocation of either strand through a nanopore during sequencing. The length of the loop-forming region may be designed to be at least 3, 4, 5, 6 and up to 20 or more nucleotides long, depending on the nanopore being used in sequencing.

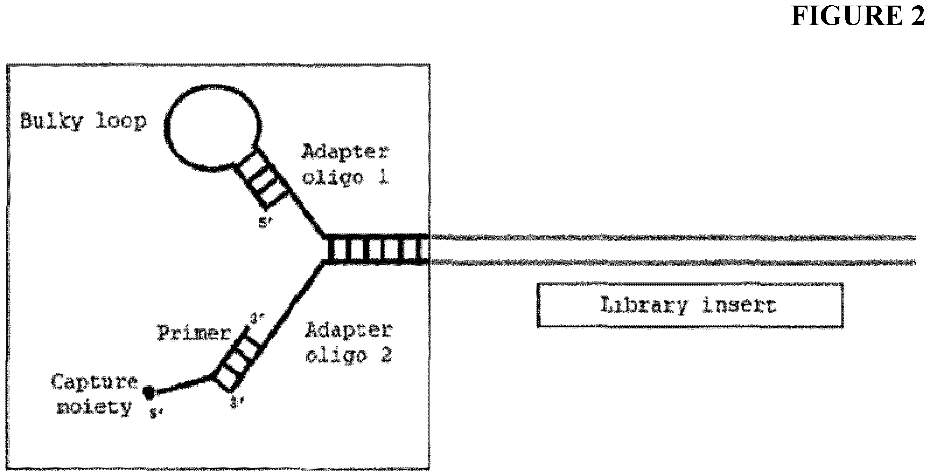

Figure 2 illustrates a different embodiment of the novel adaptor ligated to a nucleic acid of interest. Referring to Figure 2, the adaptor comprises a first strand (top strand, "Adapter oligo 1") and a second strand (bottom strand, "Adapter oligo 2"). The adaptor comprises a double-stranded region of hybridization between the first and second adaptor strands ("Adaptor oligos"). This region is capable of being ligated to a target nucleic acid ("library insert"). The first (top) strand comprises a hairpin region having a stem and a loop. The second (bottom) strand comprises a capture moiety. In the embodiment shown in Figure 2, the capture moiety is present in a third oligonucleotide hybridized to the 3'-end of the bottom adaptor strand.

A capture moiety may be any moiety capable of specifically interacting with another capture molecule. Capture moieties -capture molecule pairs include avidin (streptavidin) - biotin, antigen - antibody, magnetic (paramagnetic) particle - magnet, or oligonucleotide - complementary oligonucleotide. The capture molecule can be bound to a solid support so that any nucleic acid on which the capture moiety is present is captured on solid support and separated from the rest of the sample or reaction mixture. In some examples useful for understanding the invention and not encompassed by the wording of the claims, the capture molecule comprises a capture moiety for a secondary capture molecule. For example, a capture moiety may be an oligonucleotide complementary to a capture oligonucleotide (capture molecule). The capture oligonucleotide may be biotinylated and captured on a streptavidin bead.

In some examples useful for understanding the invention and not encompassed by the wording of the claims, the adaptor-ligated nucleic acid is enriched via capturing the capture moiety and separating the adaptor-ligated target nucleic acids from unligated nucleic acids in the sample.

In some examples useful for understanding the invention and not encompassed by the wording of the claims, the third oligonucleotide hybridized to the 3'-end of the bottom adaptor strand (Figure 2) serves as a sequencing primer or an amplification primer. In some examples useful for understanding the invention and not encompassed by the wording of the claims, the extension product of the third oligonucleotide is captured via the capture moiety. Capture of the extension product separates the extension product from unligated sample nucleic acids and optionally, from the target nucleic acids strands not having the capture moiety as well.

In some examples useful for understanding the invention and not encompassed by the wording of the claims, the stem portion of the adaptor includes a modified nucleotide increasing the melting temperature of the capture oligonucleotide, e.g., 5-methyl cytosine, 2,6-diaminopurine, 5-hydroxybutynl-2'-deoxyuridine, 8-aza-7-deazaguanosine, a ribonucleotide, a 2'O-methyl ribonucleotide or a locked nucleic acid. In another aspect, the capture oligonucleotide is modified to inhibit digestion by a nuclease, e.g., by a phosphorothioate nucleotide.

In some examples useful for understanding the invention and not encompassed by the wording of the claims an amplification step is involved, e.g., prior to ligating novel adaptors described herein. The primers may be target-specific. A target specific primer comprises at least a portion that is complementary to the target. If additional sequences are present, such as a barcode or a second primer binding site, they are typically located in the 5'-portion of the primer. The target may be a gene sequence (coding or noncoding) or a regulatory sequence present in RNA such as an enhancer or a promoter. The target may also be an inter-genic sequence. In other examples useful for understanding the invention and not encompassed by the wording of the claims, the primers are universal, e.g., can amplify all nucleic acids in the sample regardless of the target sequence. Universal primers anneal to universal primer binding sites added to the nucleic acids in the sample by extending a primer having the universal primer binding site or by ligating an adaptor, including the adaptor having the novel structures described herein.

In some embodiments, the invention utilizes a barcode. Detecting individual molecules typically requires molecular barcodes such as described in U.S. Patent Nos. 7,393,665, 8,168,385, 8,481,292, 8,685,678, and 8,722,368. A unique molecular barcode is a short artificial sequence added to each molecule in the patient's sample typically during the earliest steps of *in vitro* manipulations. The barcode marks the molecule and its progeny. The unique molecular barcode (UID) has multiple uses. Barcodes allow tracking each individual nucleic acid molecule in the sample to assess, *e.g.,* the presence and amount of circulating tumor DNA (ctDNA) molecules in a patient's blood in order to detect and monitor cancer without a biopsy (Newman, A., et al., (2014) An ultrasensitive method for quantitating circulating tumor DNA with broad patient coverage, Nature Medicine doi:10.1038/nm.3519).

A barcode can be a multiplex sample ID (MID) used to identify the source of the sample where samples are mixed (multiplexed). The barcode may also serve as a unique molecular ID (UID) used to identify each original molecule and its progeny. The barcode may also be a combination of a UID and an MID. In some embodiments, a single barcode is used as both UID and MID. In some embodiments, each barcode comprises a predefined sequence. In other embodiments, the barcode comprises a random sequence. In some examples useful for understanding the invention and not encompassed by the wording of the claims, the barcodes are between about 4-20 bases long so that between 96 and 384 different adaptors, each with a different pair of identical barcodes are added to a human genomic sample. A person of ordinary skill would recognize that the number of barcodes depends on the complexity of the sample (*i.e.,* expected number of unique target molecules) and would be able to create a suitable number of barcodes for each experiment.

Unique molecular barcodes can also be used for molecular counting and sequencing error correction. The entire progeny of a single target molecule is marked with the same barcode and forms a barcoded family. A variation in the sequence not shared by all members of the barcoded family is discarded as an artifact and not a true mutation. Barcodes can also be used for positional deduplication and target quantification, as the entire family represents a single molecule in the original sample (Newman, A., et al., (2016) Integrated digital error suppression for improved detection of circulating tumor DNA, Nature Biotechnology 34:547).

In some examples useful for understanding the invention and not encompassed by the wording of the claims, the number of UIDs in the plurality of adaptors may exceed the number of nucleic acids in the plurality of nucleic acids. In some embodiments, the number of nucleic acids in the plurality of nucleic acids exceeds the number of UIDs in the plurality of adaptors.

In some examples useful for understanding the invention and not encompassed by the wording of the claims, the invention is a library of target nucleic acids formed as described herein. The library comprises double-stranded nucleic acid molecules comprising nucleic acid targets present in the original sample. The nucleic acid molecules of the library further comprise novel adaptors described herein at one or both ends of the target nucleic acid sequence. The library nucleic acids may comprise additional elements such as barcodes and primer binding sites. In some examples useful for understanding the invention and not encompassed by the wording of the claims, the additional elements are present in adaptors and are added to the library nucleic acids via adaptor ligation. In other examples useful for understanding the invention and not encompassed by the wording of the claims, some or all of the additional elements are present in amplification primers and are added to the library nucleic acids prior to adaptor ligation by extension of the primers. The amplification may be linear (including only one round of extension) or exponential, e.g., Polymerase Chain Reaction (PCR). In some examples useful for understanding the invention and not encompassed by the wording of the claims, some additional elements are added by primer extension while the remaining additional elements are added by adaptor ligation.

The utility of adaptors and amplification primers for introducing additional elements in to a library of nucleic acids to be sequenced has been described e.g., in U.S. Patent Nos. 9476095, 9260753, 8822150, 8563478, 7741463, 8182989 and 8053192.

In some examples useful for understanding the invention and not encompassed by the wording of the claims, a step of enriching for desired target nucleic acids is comprised. The desired nucleic acids can be enriched prior to forming a library according to the novel library forming method of described herein. Alternatively, the enrichment can take place after eh library is formed, i.e., on the molecules of the library.

In some examples useful for understanding the invention and not encompassed by the wording of the claims, the method utilizes a pool of target-specific oligonucleotide probes (e.g., capture probes). The enrichment can be by subtraction in which case, capture probes are complementary to an abundant undesired sequences including ribosomal RNA (rRNA) or abundantly expressed genes (e.g., globin). In the case of subtraction, the undesired sequences are captured by the capture probes and removed from the mixture of target nucleic acids or the library of nucleic acids and discarded. For example, the capture probes may comprise a binding moiety that can be captured on solid support.

In other examples useful for understanding the invention and not encompassed by the wording of the claims, the enrichment is capture and retention in which case, capture probes are complementary to one or more target sequences. In this case the target sequences are captured by the capture probes from the mixture of target nucleic acids or the library of nucleic acids and retained while the remainder of the solution is discarded.

For enrichment, the capture probes may be free in solution or fixed to solid support. The prp0bes can be produced and amplified e.g., by the method described in the U.S. Patent 9,790,543. The probes may also comprise a binding moiety (e.g., biotin) and be capable of being captured on solid support (e.g., avidin or streptavidin containing support material).

Some examples useful for understanding the invention and not encompassed by the wording of the claims comprise intermediate purification steps. For example, any unused oligonucleotides such as excess primers and excess adaptors are removed, *e.g.,* by a size selection method selected from gel electrophoresis, affinity chromatography and size exclusion chromatography. In some examples useful for understanding the invention and not encompassed by the wording of the claims, size selection can be performed using Solid Phase Reversible Immobilization (SPRI) technology from Beckman Coulter (Brea, Cal.). In some examples useful for understanding the invention and not encompassed by the wording of the claims, a capture moiety (Figure 2) is used to capture and separate adaptor-ligated nucleic acids from unligated nucleic acids or primer extension products from the template strands.

The nucleic acids and libraries of nucleic acids formed as described herein or amplicons thereof can be subjected to nucleic acid sequencing. Sequencing can be performed by any method known in the art. Especially advantageous is the high-throughput single molecule sequencing method utilizing nanopores. In some examples useful for understanding the invention and not encompassed by the wording of the claims, the nucleic acids and libraries of nucleic acids formed as described herein are sequenced by a method involving threading through a biological nanopore (US10337060) or a solid-state nanopore (US10288599, US20180038001, US10364507). In other examples useful for understanding the invention and not encompassed by the wording of the claims, sequencing involves threading tags through a nanopore. (US8461854) or any other presently existing or future DNA sequencing technology utilizing nanopores.

In some examples useful for understanding the invention and not encompassed by the wording of the claims, the sequencing step involves sequence analysis. In some examples useful for understanding the invention and not encompassed by the wording of the claims, the analysis includes a step of sequence aligning. In some examples useful for understanding the invention and not encompassed by the wording of the claims, aligning is used to determine a consensus sequence from a plurality of sequences, *e.g.,* a plurality having the same barcodes (UID). In some examples useful for understanding the invention and not encompassed by the wording of the claims barcodes (UIDs) are used to determine a consensus from a plurality of sequences all having an identical barcode (UID). In other examples useful for understanding the invention and not encompassed by the wording of the claims, barcodes (UIDs) are used to eliminate artifacts, *i.e.,* variations existing in some but not all sequences having an identical barcode (UID). Such artifacts resulting from PCR errors or sequencing errors can be eliminated.

In some examples useful for understanding the invention and not encompassed by the wording of the claims, the number of each sequence in the sample can be quantified by quantifying relative numbers of sequences with each barcode (UID) in the sample. Each UID represents a single molecule in the original sample and counting different UIDs associated with each sequence variant can determine the fraction of each sequence in the original sample. A person skilled in the art will be able to determine the number of sequence reads necessary to determine a consensus sequence. In some examples useful for understanding the invention and not encompassed by the wording of the claims, the relevant number is reads per UID ("sequence depth") necessary for an accurate quantitative result. In some examples useful for understanding the invention and not encompassed by the wording of the claims, the desired depth is 5-50 reads per UID.

### EXAMPLES

### Example 1. Novel adaptors for nucleic acid sequencing

The sequencing adaptor is composed of two oligonucleotides (adaptor oligo 1 and adaptor oligo 2) which possess a complementary portion allowing them to form a partially double-stranded adaptor. Adapter oligo 1 further contain DNA sequined at the 5'-end which result in intramolecular stem-loop structure to form under sequencing reaction conditions. The size of the loop structure can be adjusted with DNA length and sequence composition to minimize potential of the displaced 5'-end threading through the sequencing nanopore. Adaptor oligo 2 also contains a stem-loop forming structure at the 3'-end and a free 3'-end for the nucleic acid polymerase to bind. This 3;-end is extended during amplification or sequencing with the adaptor oligo 1-ligaetd strand being eventually displaced by the sequencing or amplification polymerase, the 5'-end loop limiting threading of the displace strand through the sequencing nanopore.

### Example 2. Forming a control nucleic acid for nanopore sequencing

A DNA insert containing the desired endonuclease sites, the DNA primer annealing sites and self-complementary end sequences is first cloned in a plasmid vector. The plasmid is grown in a host bacterium; the plasmid is extracted and purified. The plasmid is then digested with an endonuclease that provides a blunt cut, for example *PmeI,* in order to linearize the plasmid. The linearized plasmid is further digested with a nicking endonuclease, for example, Nt.BbvCI to provide a short excised single stranded fragment at the 5'ends of the linearized plasmid. The plasmid is further denatured and cooled in the presence of an oligo that is complementary to the excised fragment. The excised fragment hybridizes to the complementary oligo, which may be biotin labeled to allow removal of the hybridized excised fragment using streptavidin bead purification. Excision of the 5'-ends of the plasmid allows the 3'-end to form a secondary structure such as a hairpin (stem-loop) that prevents threading of the single stranded end through a nanopore during sequencing of the DNA. The nascent 5'-ends of the plasmid are similarly designed so that on displacement during polymerase extension of a primer oligo annealed to the single stranded regions of the 3'-ends, the 5'-ends also form a secondary structure to prevent threading of the displaced 5'-ends through the nanopore.

## Claims

1. An adaptor for nucleic acid libraries comprising a first strand and a second strand wherein:
a. the first strand has a 5'-portion and a 3'-portion, wherein the 5' portion forms a stem-loop structure having a loop and a stem containing the 5'-end of the first strand, and the 3'-portion comprises a sequence complementary to the second strand;
b. the second strand has a 5'-portion and a 3'-portion, wherein the 3' portion forms a stem-loop structure having a loop and a stem containing the 3'-end of the second strand, and the 5'-portion comprises a sequence complementary to the first strand; and
c. the first and second strands form a duplex via the 3'-portion of the first strand and the 5'-portion of the second strand.

2. The adaptor of claim 1, wherein the 3'-portion of the second strand is extendable by a nucleic acid polymerase.

3. The adaptor of claim 1, wherein one or both loop-forming regions are at least 4, 5, 6 and up to 20 or more nucleotides long.

4. The adaptor of claim 1, wherein the adaptor comprises one or more molecular barcodes.

5. The adaptor of claim 4, wherein the molecular barcode is selected from a sample barcode (SID) and a unique molecular identifier barcode (UID).

6. The adaptor of claim 4, wherein the SID is located outside the duplex formed by the 3'-portion of the first strand and the 5'-portion of the second strand.

7. The adaptor of claim 4, wherein the UID is located within the duplex formed by the 3'-portion of the first strand and the 5'-portion of the second strand.

8. The adaptor of claim 4, wherein the SID and the UID comprise a predefined sequence 0r a random sequence.

9. A method of making a library of nucleic acids comprising: attaching to a plurality of double-stranded nucleic acids in a sample a plurality of adaptors, each adaptor comprising
a. the first strand having a 5'-portion and a 3'-portion, wherein the 5' portion forms a stem-loop structure having a loop and a stem containing the 5'-end of the first strand, and the 3'-portion comprises a sequence complementary to the second strand;
b. the second strand having a 5'-portion and a 3'-portion, wherein the 3' portion forms a stem-loop structure having a loop and a stem containing an extendable 3'-end of the second strand, and the 5'-portion comprises a sequence complementary to the first strand; and
c. the first and second strands forming a duplex via the 3'-portion of the first strand and the 5'-portion of the second strand.

10. The method of claim 9, wherein the adaptor is attached via ligating the duplex formed the 3'-portion of the first strand and the 5'-portion of the second strand to one or both ends of the double-stranded nucleic acids.

11. The method of claim 9, wherein prior to attaching, the plurality of nucleic acids is pre-treated to form a blunt end at one or both ends of each nucleic acid.

12. The method of claim 9, wherein the duplex formed by the 3'-portion of the first strand and the 5'-portion of the second strand has a single stranded overhang of one or more nucleotides.

13. A method of sequencing nucleic acids in a sample, the method comprising forming a library of nucleic acids according to claim 9 and sequencing the library by a sequencing by synthesis method comprising extending the extendable 3'-end of the second strand of the adaptor.

14. A control nucleic acid for use in a sequencing reaction comprising a first strand and a second strand complementary to the first strand and two termini, wherein at least one or the two termini comprises:
a. a 3'-overhang forming a stem-loop structure, wherein the 3'-end of the overhang is extendable by a nucleic acid polymerase, and
b. a 5'-end which upon displacement by the extended 3'-end, forms a stem-loop structure.

15. A method of sequencing a library of nucleic acids comprising contacting the library with a control nucleic acid according to claim 14 and sequencing the library of nucleic acid by a method including detection with a nanopore.

## Patentansprüche

1. Adaptor für Nukleinsäurebibliotheken, umfassend einen ersten Strang und einen zweiten Strang, wobei:
a. der erste Strang einen 5'-Abschnitt und einen 3'-Abschnitt aufweist, wobei der 5'-Abschnitt eine Stamm-Schleife-Struktur mit einer Schleife und einem Stamm, der das 5'-Ende des ersten Stranges enthält, bildet, und der 3'-Abschnitt eine Sequenz umfasst, die komplementär ist zu dem zweiten Strang;
b. der zweite Strang einen 5'-Abschnitt und einen 3'-Abschnitt aufweist, wobei der 3'-Abschnitt eine Stamm-Schleife-Struktur mit einer Schleife und einem Stamm, der das 3'-Ende des zweiten Stranges enthält, bildet, und der 5'-Abschnitt eine Sequenz umfasst, die komplementär ist zu dem ersten Strang; und
c. der erste und zweite Strang über den 3'-Abschnitt des ersten Stranges und den 5'-Abschnitt des zweiten Stranges einen Doppelstrang bilden.

2. Adaptor nach Anspruch 1, wobei der 3'-Abschnitt des zweiten Stranges durch eine Nukleinsäurepolymerase verlängert werden kann.

3. Adaptor nach Anspruch 1, wobei eine oder beide schleifenbildende(n) Region(en) mindestens 4, 5, 6 und bis zu 20 oder mehr Nukleotide lang ist/sind.

4. Adaptor nach Anspruch 1, wobei der Adaptor einen oder mehrere molekulare(n) Barcode(s) umfasst.

5. Adaptor nach Anspruch 4, wobei der molekulare Barcode aus einem Proben-Barcode (SID) und einem eindeutigen molekularen Kennungs-Barcode (UID) ausgewählt ist.

6. Adaptor nach Anspruch 4, wobei sich der SID außerhalb des von dem 3'-Abschnitt des ersten Stranges und dem 5'-Abschnitt des zweiten Stranges gebildeten Doppelstranges befindet.

7. Adaptor nach Anspruch 4, wobei sich der UID innerhalb des von dem 3'-Abschnitt des ersten Stranges und dem 5'-Abschnitt des zweiten Stranges gebildeten Doppelstranges befindet.

8. Adaptor nach Anspruch 4, wobei der SID und der UID eine vordefinierte Sequenz oder eine zufällige Sequenz umfassen.

9. Verfahren zum Erstellen einer Bibliothek von Nukleinsäuren, umfassend: Verknüpfen einer Vielzahl von Adaptoren mit einer Vielzahl von doppelsträngigen Nukleinsäuren in einer Probe, wobei jeder Adaptor Folgendes umfasst
a. den ersten Strang mit einem 5'-Abschnitt und einem 3'-Abschnitt, wobei der 5'-Abschnitt eine Stamm-Schleife-Struktur mit einer Schleife und einem Stamm, der das 5'-Ende des ersten Stranges enthält, bildet, und der 3'-Abschnitt eine Sequenz umfasst, die komplementär ist zu dem zweiten Strang;
b. den zweiten Strang mit einem 5'-Abschnitt und einem 3'-Abschnitt, wobei der 3'-Abschnitt eine Stamm-Schleife-Struktur mit einer Schleife und einem Stamm, der ein verlängerbares 3'-Ende des zweiten Stranges enthält, bildet, und der 5'-Abschnitt eine Sequenz umfasst, die komplementär ist zu dem ersten Strang; und
c. den ersten und zweiten Strang, die über den 3'-Abschnitt des ersten Stranges und den 5'-Abschnitt des zweiten Stranges einen Doppelstrang bilden.

10. Verfahren nach Anspruch 9, wobei der Adaptor über Ligieren des von dem 3'-Abschnitt des ersten Stranges und dem 5'-Abschnitt des zweiten Stranges gebildeten Doppelstranges mit einem oder beiden Ende(n) der doppelsträngigen Nukleinsäuren verknüpft wird.

11. Verfahren nach Anspruch 9, wobei vor dem Verknüpfen die Vielzahl von Nukleinsäuren unter Bildung eines stumpfen Endes an einem oder beiden Ende(n) jeder Nukleinsäure vorbehandelt wird.

12. Verfahren nach Anspruch 9, wobei der von dem 3'-Abschnitt des ersten Stranges und dem 5'-Abschnitt des zweiten Stranges gebildete Doppelstrang einen einzelsträngigen Überhang von einem oder mehreren Nukleotid(en) aufweist.

13. Verfahren zum Sequenzieren von Nukleinsäuren in einer Probe, wobei das Verfahren das Bilden einer Bibliothek von Nukleinsäuren nach Anspruch 9 und das Sequenzieren der Bibliothek mittels eines Sequencing-by-Synthesis-Verfahrens, umfassend das Verlängern des verlängerbaren 3'-Endes des zweiten Stranges des Adaptors, umfasst.

14. Kontrollnukleinsäure zur Verwendung in einer Sequenzierungsreaktion, umfassend einen ersten Strang und einen zweiten Strang, der komplementär ist zu dem ersten Strang, und zwei Termini, wobei mindestens einer der oder beide Termini Folgendes umfasst/umfassen:
a. einen 3'-Überhang, der eine Stamm-Schleife-Struktur bildet, wobei das 3'-Ende des Überhanges durch eine Nukleinsäurepolymerase verlängert werden kann, und
b. ein 5'-Ende, das bei Verdrängung durch das verlängerte 3'-Ende eine Stamm-Schleife-Struktur bildet.

15. Verfahren zum Sequenzieren einer Bibliothek von Nukleinsäuren, umfassend das Inkontaktbringen der Bibliothek mit einer Kontrollnukleinsäure nach Anspruch 14 und das Sequenzieren der Bibliothek von Nukleinsäuren mittels eines Verfahrens, das den Nachweis mit einer Nanopore einschließt.

## Revendications

1. Adaptateur pour des banques d'acides nucléiques comprenant un premier brin et un second brin dans lequel :
a. le premier brin a une partie 5' et une partie 3', dans lequel la partie 5' forme une structure tige-boucle ayant une boucle et une tige contenant l'extrémité 5' du premier brin, et la partie 3' comprend une séquence complémentaire du second brin ;
b. le second brin a une partie 5' et une partie 3', dans lequel la partie 3' forme une structure tige-boucle ayant une boucle et une tige contenant l'extrémité 3' du second brin, et la partie 5' comprend une séquence complémentaire du premier brin ; et
c. les premier et second brins forment un duplex par le biais de la partie 3' du premier brin et de la partie 5' du second brin.

2. Adaptateur selon la revendication 1, dans lequel la partie 3' du second brin est extensible par une acide nucléique polymérase.

3. Adaptateur selon la revendication 1, dans lequel une région formant une boucle ou les deux sont longues d'au moins 4, 5, 6 et jusqu'à 20 nucléotides ou plus.

4. Adaptateur selon la revendication 1, dans lequel l'adaptateur comprend un ou plusieurs codes-barres moléculaires.

5. Adaptateur selon la revendication 4, dans lequel le code-barres moléculaire est choisi parmi un code-barres d'échantillon (SID) et un code-barres d'identifiant moléculaire unique (UID).

6. Adaptateur selon la revendication 4, dans lequel le SID est situé à l'extérieur du duplex formé par la partie 3' du premier brin et la partie 5' du second brin.

7. Adaptateur selon la revendication 4, dans lequel l'UID est situé à l'intérieur du duplex formé par la partie 3' du premier brin et la partie 5' du second brin.

8. Adaptateur selon la revendication 4, dans lequel le SID et l'UID comprennent une séquence prédéfinie ou une séquence aléatoire.

9. Procédé de préparation d'une banque d'acides nucléiques comprenant : la fixation à une pluralité d'acides nucléiques bicaténaires dans un échantillon d'une pluralité d'adaptateurs, chaque adaptateur comprenant
a. le premier brin ayant une partie 5' et une partie 3', dans lequel la partie 5' forme une structure tige-boucle ayant une boucle et une tige contenant l'extrémité 5' du premier brin, et la partie 3' comprend une séquence complémentaire du second brin ;
b. le second brin ayant une partie 5' et une partie 3', dans lequel la partie 3' forme une structure tige-boucle ayant une boucle et une tige contenant une extrémité 3' extensible du second brin, et la partie 5' comprend une séquence complémentaire du premier brin ; et
c. les premier et second brins formant un duplex par le biais de la partie 3' du premier brin et de la partie 5' du second brin.

10. Procédé selon la revendication 9, dans lequel l'adaptateur est fixé par le biais d'une ligature du duplex formé par la partie 3' du premier brin et la partie 5' du second brin à une ou aux deux extrémités des acides nucléiques bicaténaires.

11. Procédé selon la revendication 9, dans lequel avant la fixation, la pluralité d'acides nucléiques est prétraitée pour former une extrémité franche au niveau d'une ou des deux extrémités de chaque acide nucléique.

12. Procédé selon la revendication 9, dans lequel le duplex formé par la partie 3' du premier brin et la partie 5' du second brin a un surplomb monocaténaire d'un ou de plusieurs nucléotides.

13. Procédé de séquençage d'acides nucléiques dans un échantillon, le procédé comprenant la formation d'une banque d'acides nucléiques selon la revendication 9 et le séquençage de la banque par un séquençage par un procédé de synthèse comprenant l'extension de l'extrémité 3' extensible du second brin de l'adaptateur.

14. Acide nucléique de contrôle pour une utilisation dans une réaction de séquençage comprenant un premier brin et un second brin complémentaire du premier brin et deux extrémités terminales, dans lequel au moins une ou les deux extrémités terminales comprend :
a. un surplomb 3' formant une structure tige-boucle, dans lequel l'extrémité 3' du surplomb est extensible par une acide nucléique polymérase, et
b. une extrémité 5' qui lors du déplacement par l'extrémité 3' étendue, forme une structure tige-boucle.

15. Procédé de séquençage d'une banque d'acides nucléiques comprenant la mise en contact de la banque avec un acide nucléique de contrôle selon la revendication 14 et le séquençage de la banque d'acides nucléiques par un procédé comprenant la détection avec un nanopore.
